# EUROPÄISCHE PATENTSCHRIFT

(11) **EP 0 585 766 B1**
(45) Veröffentlichungstag und Bekanntmachung des Hinweises auf die Patenterteilung: **22.04.1998**
(21) Anmeldenummer: 93113419.1
(22) Anmeldetag: 23.08.1993
(51) Int. Cl.: A61F 13/15

(54) **Absorbierendes wegwerfhöschen**
Disposable absorbent pant
Slip absorbant à jeter

(30) Priorität: 24.08.1992 JP 59281/92
(43) Veröffentlichungstag der Anmeldung: 09.03.1994
(73) Patentinhaber: UNI-CHARM CORPORATION, Kawanoe-shi Ehime-ken (JP)
(72) Erfinder: Fujioka, Yoshihisa, Mitoyo-gun, Kagawa-ken (JP); Mukai, Hirotomo, Kawanoe-shi, Ehime-ken (JP)
(74) Vertreter: Sperling, Rüdiger, Dipl.-Ing.

(56) Entgegenhaltungen:
- EP-A- 0 187 728
- FR-A- 2 517 525
- US-A- 2 654 367
- US-A- 3 225 918

## Beschreibung

Die vorliegende Erfindung betrifft absorbierende Höschen, wie zum Beispiel Wegwerfwindeln nach dem Oberbegriff des Anspruchs 1.

Wegwerfwindeln des Höschentyps sind beispielsweise aus der japanischen Patentveröffentlichung Nr. 1992-42017 bekannt. Wie Fig. 10 und 11 dieser Patentveröffentlichung zeigen, umfaßt diese Windel nach dem Stand der Technik einen Schichtkörper, der aus einer flüssigkeitsdurchlässigen Decklage, die so zugeschnitten ist, daß sie in seitlicher Richtung länger ist, einer flüssigkeitsundurchlässigen Außenlage, die ebenfalls so zugeschnitten ist, daß sie in seitlicher Richtung länger ist, und einer zwischen diese gelegten flüssigkeitsabsorbierenden Platte besteht, sowie aus einem um einen der in Längsrichtung einander gegenüberliegenden Ränder des Schichtkörpers um eine Hüftöffnung verlaufenden elastischen Element sowie entlang dem anderen Rand um jeweilige Beinöffnungen verlaufenden elastischen Elementen besteht, wobei der Schichtkörper in Querrichtung übereinander gefaltet wird, die Ränder des dergestalt gefalteten Schichtkörpers gegenüber der Faltung miteinander verbunden werden und Ränder von Ausschnitten, die in Mittelpositionen des unteren Endes des Schichtkörpers ausgebildet sind, ebenfalls miteinander verbunden werden, um so einen Schrittbereich zu bilden.

Während die in vorstehend bezeichneter Patentschrift aufgezeigte Windel eine gute Paßform im Schritt des Trägers bietet, da die Windel im Schrittbereich mit Ausschnitten versehen ist, um so sicherzustellen, daß die Beinöffnungen unterhalb der Unterfläche des Schrittbereiches angeordnet sind, weist ein zentraler Bereich des Schrittbereiches keine flüssigkeitsabsorbierende Platte auf und es ist daher schwierig, den Austritt von flüssigen Ausscheidungen entlang den einander seitlich gegenüberliegenden Seiten des Schrittbereiches zu verhindern.

Aus der FR-A-2,517,525 ist ein absorbierendes Wegwerfhöschen mit einem Vorder- und einem Hinterteil bekannt, die jeweils eine flüssigkeitsdurchlässige Decklage, eine flüssigkeitsundurchlässige Außenlage und eine zwischen diese gelegte flüssigkeitsabsorbierende Platte umfassen. Die Vorder- und Hinterteile sind so zusammengesetzt, daß sie eine Grundstruktur des Höschens mit einer Hüftöffnung und zwei Beinöffnungen bilden, um die herum dehnbare elastische Elemente angeordnet sind. Die Vorder- und Hinterteile sind an ihren seitlich entgegengesetzten Seitenrändern und im Schrittbereich miteinander verbunden. Eine analog dem Schrittbereich in U-Form gekrümmte flüssigkeitsabsorbierende Hilfsplatte ist wenigstens an den oberen Enden ihrer U-Form im Schrittbereich an der Innenfläche der Grundstruktur befestigt.

Auf Grund der Verbindungsstelle, wie z.B. durch eine Schweißnaht, im Schrittbereich des Windelhöschens besteht die Gefahr, daß Körperflüssigkeit abwärts sickert und diese Verbindungsstelle durchdringt oder an ihr entlang nach außen läuft, so daß über dem Höschen getragene Kleidung naß wird. Dadurch wird sowohl der Tragekomfort und die Sicherheit des Höschens eingeschränkt als auch eine Gefahr für häufiges Verschmutzen der übrigen Kleidung begründet.

Der vorliegenden Erfindung liegt daher die Aufgabe zu Grunde, eine Wegwerfwindel zu schaffen, die eine hohe Auslaufsicherheit aufweist.

Dies kann bei einem absorbierenden Wegwerfhöschen, wie es der Fachmann der FR-A-2,517,525 entnehmen kann, gemäß dem Anspruch 1 dadurch realisiert werden, daß die Hilfsplatte so an der Grundstruktur befestigt ist, daß ihre äußere Unterseite in einem Abstand oberhalb der Oberfläche des Schrittbereiches liegt.

Durch die Erfindung wird somit ein absorbierendes Wegwerfhöschen mit einem Vorder- und einem Hinterteil geschaffen, die so zusammengesetzt sind, daß sie ein Höschen mit einer Hüftöffnung und zwei Beinöffnungen bilden, und jeweils aus einer flüssigkeitsdurchlässigen Decklage, einer flüssigkeitsundurchlässigen Außenlage, einer zwischen diese gelegten flüssigkeitsabsorbierenden Platte und in Umfangsrichtung der Hüftöffnung und der Beinoffnungen angeordneten und dehnbaren elastischen Elementen bestehen, wobei die Vorder- und Hinterteile an ihren seitlich entgegengesetzten Seitenrändern und im Schrittbereich miteinander verbunden sind, wodurch eine Grundstuktur des Höschens gebildet wird, und wobei eine analog dem Schrittbereich in U-Form gekrümmte flüssigkeitsabsorbierende Hilfsplatte wenigstens an den oberen Enden ihrer U-Form im Schrittbereich an der Innenfläche der Grundstruktur befestigt ist, wobei ferner die Hilfsplatte so an der Grundstruktur befestigt ist, daß ihre äußere Unterseite in einem Abstand oberhalb der Oberfläche des Schrittbereiches liegt.

Dadurch wird erreicht, daß gerade in dem Schrittbereich Körperflüssigkeit nicht ungehindert auf die Schweißnaht und von dort nach außerhalb der Windel gelangen kann, sondern von der Hilfsplatte so aufgenommen werden kann, und daß die ggf. flüssigkeitsgetränkte Hilfsplatte nicht direkt mit der Schweißlinie oder -naht in Kontakt kommt, auch wenn sie bedingt durch eine Flüssigkeitsaufnahme aufgequollen oder schwerer ist und dadurch weiter nach unten reicht. Es wird sozusagen ein Sicherheitsvolumen vorgesehen, um zu verhindern, daß Flüssigkeit an die Schweißnaht oder allgemein Verbindungsstelle im Schrittbereich gelangen und diese durchdringen oder daran entlangfließen kann.

Vorteilhafte und daher bevorzugte Weiterbildungen der Erfindung ergeben sich aus den abhängigen Ansprüchen und deren Kombinationen.

Vorzugsweise ist der durch die konvex gekrümmte Schweißlinie bestimmte Schrittbereich entlang seinen äußeren Rändern mit einem entsprechend gekrümmten Ausschnitt versehen.

Bei dem gemäß der Erfindung wie vorstehend dargelegt aufgebauten Höschen ist der Schrittbereich zuverlässig in der Mitte im Schritt des Trägers ausgerichtet und die einander seitlich gegenüberliegenden Seiten des Schrittbereiches bedecken wenigstens jeweils die Innenseite der Oberschenkel des Trägers. Der Schrittbereich enthält auch die flüssigkeitsabsorbierende Platte und daher werden flüssige Ausscheidungen auch in diesem Bereich effektiv absorbiert.

### KURZBESCHREIBUNG DER FIGUREN

Die Erfindung wird anhand eines Beispiels unter Bezug auf die beiliegenden Figuren erläutert, wobei
- Fig. 1: eine perspektivische Darstellung ist, die eine Ausführungsform einer gemäß der Lehre der Erfindung aufgebauten Windel zeigt; und
- Fig. 2: eine Schnittdarstellung entlang einer Linie X-X in Fig. 1 ist.

### BESCHREIBUNG DER BEVORZUGTEN AUSFÜHRUNGSFORM

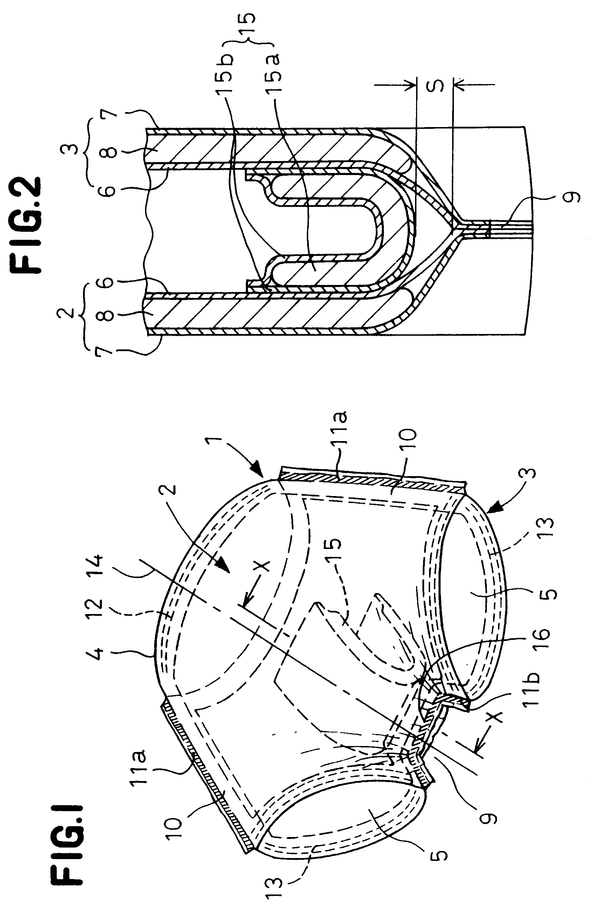

Wie Fig. 1 und 2 zeigen, umfaßt die Grundstruktur 1 der Windel allgemein einen Vorderteil 2 und einen Hinterteil 3, die in Form eines Höschens mit einer Hüftöffnung 4 und zwei Beinöffnungen 5 zusammengesetzt sind. Der Vorderteil 2 und der Hinterteil 3 umfassen jeweils eine flüssigkeitsdurchlässige Decklage 6, eine flüssigkeitsundurchlässige Außenlage 7 und eine zwischen die Deck- und die Außenlage 6, 7 gelegte, flüssigkeitsabsorbierende Platte 8.

Die unteren Enden des Vorderteils 2 und des Hinterteils 3 sind in der Mittelposition mit Ausschnitten 9 versehen, die einen Schrittbereich bilden. Der Vorderteil 2 und der Hinterteil 3 sind entlang thermischen oder Ultraschall-Schweißlinien 11a, 11b, die jeweils parallel zu den Ausschnitten 9 und den seitlich gegenüberliegenden Seitenrändern 10 verlaufen, miteinander verschweißt, so daß schmale Ränder dieser Ausschnitte 9 und der einander gegenüberliegenden Seitenränder unverschweißt belassen werden. Größe, Form und Krümmungsradius der Schweißlinie 11b kann in Abhängigkeit davon, ob die Windel für einen Erwachsenen oder für ein Baby bestimmt ist, in geeigneter Weise gewählt werden, sofern die Schweißlinie 11b konvex auf die Hüftlinie des Vorderteils 2 und des Hinterteils 3 zu gekrümmt ist und wenigstens die Innenseiten der jeweiligen Beinöffnungen 5 sich über einen Scheitelpunkt 16 der konvex gekrümmten Schweißlinie 11b nach unten erstrecken.

In Umfangsrichtung dehnbare elastische Elemente 12, 13 sind zwischen die jeweiligen Ränder von Deck- und Außenlage 6, 7 gelegt, die sich jeweils um eine Hüftöffnung und die Beinöffnungen über die Platte 8 hinaus erstrecken, und die Ränder sind miteinander durch durch Heißschmelzkleber oder Schweißeinrichtungen verbunden. Die über die Platte 8 hinausragenden, einander seitlich gegenüberliegenden Seitenränder der Deck- und der Außenlage 6, 7 sind ebenfalls in ähnlicher Weise miteinander verbunden.

Die einander seitlich gegenüberliegenden Seitenränder und die unteren Ränder um die jeweiligen Beinöffnungen des Vorderteils und des Hinterteils sind so dargestellt, daß sie weder parallel noch senkrecht zu einer vertikalen Achse 14 verlaufen. Diese Seitenränder können innerhalb des Umfanges der Erfindung parallel zur vertikalen Achse 14 und diese unteren Ränder um die jeweiligen Beinöffnungen senkrecht zur vertikalen Achse 14 verlaufen.

Die Grundstruktur 1 der Windel ist auf der Oberfläche ihres Schrittbereiches mit einer flüssigkeitsabsorbierenden Hilfsplatte 15 versehen, die in Längsrichtung der Grundstruktur 1 der Windel eine längliche Form hat. Die Hilfsplatte 15 umfaßt einen mit flüssigkeitsdurchlässigen Lagen 15b bedeckten flüssigkeitsabsorbierenden Kern 15a und ist wenigstens an ihren in Längsrichtung entgegengesetzten Enden mit der Oberfläche der Decklage 4 mittels Klebstoffes verbunden, so daß sie in U-Form gekrümmt ist. Wenn die Grundstruktur 1 der Windel einem Träger angelegt ist, ist die Hilfsplatte 15 zusammen mit der Grundstruktur 1 der Windel in U-Form gekrümmt. Die Hilfsplatte 15 ist vorzugsweise in einer Weise mit der Grundstruktur 1 der Windel verbunden, daß die dergestalt gekrümmte Hilfsplatte 15 schwimmend über der Oberfläche des Schrittbereiches der Grundstruktur liegt, und genauer ist die gekrümmte Hilfplatte 15 mit ihrer äußeren Unterseite mit einem Abstand S von wenigstens 10 mm von der Oberfläche des Schrittbereiches der Grundstruktur beabstandet. Es ist jedoch auch innerhalb des Umfanges der Erfindung möglich, die Hilfsplatte 15 so anzuordnen, daß die Unterfläche der Hilfsplatte 15 auch in gekrümmtem Zustand mit dem Schrittbereich der Windel in Berührung bleibt. Die untere Lage der Hilfsplatte 15 kann eine flüssigkeitsundurchlässige Lage umfassen, und die Hilfsplatte 15 kann dehnbare Seitenklappen aufweisen, die sich von den seitlich gegenüberliegenden Seiten derselben erstrecken, ohne vom Umfang der Erfindung abzuweichen.

Die Bestandteile der Windel 1 können aus allgemein in bekannten Windeln verwendeten Materialien hergestellt sein. Beispielsweise kann die Decklage 6 aus Vliesstoff, die Außenlage 7 aus Kunststoffolie, die Platte 8 aus Faserpulpe, gemischt mit hochabsorbierendem Polymer, hergestellt sein, und die elastischen Elemente 12, 13 können aus natürlichem oder synthetischem Gummi hergestellt sein. Der Kern 15a der Platte 15 kann aus Faserpulpe, gemischt mit hochabsorbierendem Polymer, und die Lage 15b, die den Kern 15a bedeckt, kann aus Vliesstoff, vorzugsweise dehnbarem Vliesstoff hergestellt sein.

Bei der wie vorstehend beschrieben gemäß der Erfindung aufgebauten Windel ermöglicht es das Anordnen der flüssigkeitsabsorbierenden Hilfsplatte über dem Schrittbereich, daß der Schrittbereich flüssige Ausscheidungen ausreichend aufnimmt, um ein Austreten von flüssigen Ausscheidungen entlang den einander seitlich gegenüberliegenden Seitenrändern des Schrittbereiches zu verhindern.

## Patentansprüche

1. Absorbierendes Wegwerfhöschen mit einem Vorder- (2) und einem Hinterteil (3), die so zusammengesetzt sind, daß sie ein Höschen mit einer Hüftöffnung (4) und zwei Beinöffnungen (5) bilden, und jeweils aus einer flüssigkeitsdurchlässigen Decklage (6), einer flüssigkeitsundurchlässigen Außenlage (7), einer zwischen diese gelegten flüssigkeitsabsorbierenden Platte (8) und in Umfangsrichtung der Hüftöffnung (4) und der Beinöffnungen (5) angeordneten und dehnbaren elastischen Elementen (12, 13) bestehen, wobei die Vorder- und Hinterteile (2 bzw. 3) an ihren seitlich entgegengesetzten Seitenrändern (10) und im Schrittbereich miteinander verbunden sind, wodurch eine Grundstuktur des Höschens gebildet wird, und wobei eine analog dem Schrittbereich in U-Form gekrümmte flüssigkeitsabsorbierende Hilfsplatte (15) wenigstens an den oberen Enden ihrer U-Form im Schrittbereich an der Innenfläche der Grundstruktur befestigt ist, **dadurch gekennzeichnet**, daß die Hilfsplatte (15) so an der Grundstruktur befestigt ist, daß ihre äußere Unterseite in einem Abstand (S) oberhalb der Oberfläche des Schrittbereiches liegt.

2. Absorbierendes Wegwerfhöschen nach Anspruch 1, **dadurch gekennzeichnet**, daß der Abstand (S), um den die äußere Unterseite der flüssigkeitsabsorbierende Hilfsplatte (15) oberhalb der Oberfläche des Schrittbereiches liegt, wenigstens 10 mm beträgt.

3. Absorbierendes Wegwerfhöschen nach Anspruch 1 oder 2, **dadurch gekennzeichnet**, daß die Hilfsplatte (15) eine flüssigkeitsundurchlässige untere Lage enthält.

4. Absorbierendes Wegwerfhöschen nach einem der vorhergehenden Ansprüche, **dadurch gekennzeichnet**, daß die Vorder- und Hinterteile (2 bzw. 3) nahe an unteren Enden an einer konvex in Richtung auf eine Hüftlinie des Vorder- (2) und des Hinterteils (3) zu gekrümmten Schweißlinie (11b) miteinander verschweißt sind.

5. Absorbierendes Wegwerfhöschen nach Anspruch 4, **dadurch gekennzeichnet**, daß der durch die konvex gekrümmte Schweißlinie (11b) gebildete Schrittbereich entlang seinem äußeren Rand mit einem entsprechend gekrümmten Ausschnitt (9) versehen ist.

6. Absorbierendes Wegwerfhöschen nach Anspruch 4 oder 5, **dadurch gekennzeichnet**, daß sich wenigstens die Innenseiten von jeweiligen Beinöfffnungen (10) über einen Scheitelpunkt (9) der konvex gekrümmten Schweißlinie (8) nach unten erstrecken.

7. Absorbierendes Wegwerfhöschen nach einem der vorhergehenden Ansprüche, **dadurch gekennzeichnet**, daß der Vorderteil (2) und der Hinterteil (3) getrennt voneinander geformt sind.

8. Absorbierendes Wegwerfhöschen nach Anspruch 7, **dadurch gekennzeichnet**, daß der Vorderteil (2) und der Hinterteil (3) an ihren entgegengesetzten Seiten und im Schrittbereich entlang thermischen oder Ultraschallschweißlinien miteinander verschweiß sind, insbesondere so, daß schmale Ränder unverschweißt belassen sind.

9. Absorbierendes Wegwerfhöschen nach einem der vorhergehenden Ansprüche, **dadurch gekennzeichnet**, daß die Hilfsplatte (15) dehnbare, von seitlich einander gegenüberliegenden Seiten derselben ausgehende Seitenklappen aufweist.

10. Absorbierendes Wegwerfhöschen nach einem der vorhergehenden Ansprüche, **dadurch gekennzeichnet**, daß die Decklage (6) und die Außenlage (7) nach außen über den Umfangsrand der jeweiligen Platte (8) hinausragende äußere Umfangsränder aufweisen, an denen sie insbesondere verklebt oder verschweißt sind.

## Claims

1. Disposable absorbent pants having a front part (2) and a rear part (3), which are put together in such a way that they form pants with a hip opening (4) and two leg openings (5), and in each case comprise a liquid-permeable liner (6), a liquid-impermeable outer layer (7), a liquid-absorbent sheet (8) placed between the latter and elastic elements (12, 13) which are arranged and can stretch in the circumferential direction of the hip opening (4) and the leg openings (5), the front and rear parts (2 and 3) being joined to each other at their laterally opposed side edges (10) and in the crotch region, whereby a basic structure of the pants is formed, and a liquid-absorbent auxiliary sheet (15), which is curved in a U shape analogously to the crotch region, being fastened at least at the upper ends of its U shape in the crotch region to the inner surface of the basic structure, characterized in that the auxiliary sheet (15) is fastened to the basic structure in such a way that its outer underside lies as a distance (S) above the surface of the crotch region.

2. Disposable absorbent pants according to Claim 1, characterized in that the distance (S) by which the outer underside of the liquid-absorbent auxiliary sheet (15) lies above the surface of the crotch region is at least 10 mm.

3. Disposable absorbent pants according to Claim 1 or 2, characterized in that the auxiliary sheet (15) contains a liquid-impermeable lower layer.

4. Disposable absorbent pants according to one of the preceding claims, characterized in that the front and rear parts (2 and 3) are welded to each other close to lower ends on a welding line (11b) curved convexly in the direction of a hip line of the front part (2) and of the rear part (3).

5. Disposable absorbent pants according to Claim 4, characterized in that the crotch region formed by the convexly curved welding line (11b) is provided along its outer edge with a correspondingly curved cutout (9).

6. Disposable absorbent pants according to Claim 4 or 5, characterized in that at least the inner sides of respective leg openings (10) extend downwards beyond an apex (9) of the convexly curved welding line (8).

7. Disposable absorbent pants according to one of the preceding claims, characterized in that the front part (2) and the rear part (3) are formed separately from each other.

8. Disposable absorbent pants according to Claim 7, characterized in that the front part (2) and the rear part (3) are welded to each other on their opposed sides and in the crotch region along thermal or ultrasonic welding lines, in particular in such a way that narrow edges are left unwelded.

9. Disposable absorbent pants according to one of the preceding claims, characterized in that the auxiliary sheet (15) has stretchable side flaps extending from sides of the said sheet lying laterally opposite each other.

10. Disposable absorbent pants according to one of the preceding claims, characterized in that the liner (6) and the outer layer (7) have outer peripheral edges which extend outwards beyond the peripheral edge of the respective sheet (8) and at which they are, in particular, adhesively bonded or welded.

## Revendications

1. Petite culotte jetable absorbante, comprenant une partie avant (2) et une partie arrière (3), lesquelles sont assemblées de manière à former une petite culotte avec une ouverture de hanches (4) et deux ouvertures de passage des jambes (5), et se composent, respectivement, d'une mince couche de revêtement (6) perméable aux liquides, d'une mince couche extérieure (7) imperméable aux liquides, d'une couche plus épaisse (8) absorbant les liquides, interposée entre ces deux couches, et d'éléments élastiques (12, 13) mis en place et extensibles dans le sens de la circonférence de l'ouverture de hanches (4) et des ouvertures de passage des jambes (5), petite culotte dans laquelle les parties avant (2) et arrière (3) sont jointes sur leurs bords latéraux (10) en opposition latérale et dans la zone de l'entrejambe, ce qui donne naissance à une structure de base de la petite culotte, et dans laquelle une garniture auxiliaire (15) absorbant les liquides, pliée en forme de U de manière analogue à la zone de l'entrejambe est fixée, au moins au niveau des extrémités supérieures de sa forme de U, dans la zone de l'entrejambe, à la surface intérieure de la structure de base, caractérisée en ce que la garniture auxiliaire (15) est fixée à la structure de base de telle manière que sa face inférieure extérieure se situe au-dessus et à une distance (S) de la surface de la zone d'entrejambe.

2. Petite culotte jetable absorbante selon la revendication 1, caractérisée en ce que la distance (S) dont la face inférieure extérieure de la garniture auxiliaire (15) absorbant les liquides est écartée de la surface de la zone de l'entrejambe est d'au moins 10 mm.

3. Petite culotte jetable absorbante selon l'une des revendications 1 ou 2, caractérisée en ce que la garniture auxiliaire (15) comprend une couche inférieure imperméable aux liquides.

4. Petite culotte jetable absorbante selon l'une des revendications qui précèdent, caractérisée en ce que les parties avant et arrière (2 et 3) sont soudées entre elles à proximité d'extrémités inférieures, sur une ligne de soudure (11b) s'incurvant avec une courbure convexe dans la direction d'une ligne de hanches de la partie avant (2) et de la partie arrière (3).

5. Petite culotte jetable absorbante selon la revendication 4, caractérisée en ce que la zone d'entrejambe formée par la ligne de soudure (11b) d'incurvation convexe est pourvue d'une échancrure (9) d'incurvation correspondante le long de son bord extérieur.

6. Petite culotte jetable absorbante selon l'une des revendications 4 ou 5, caractérisée en ce qu'au moins les faces inférieures d'ouvertures de passage de jambes (10) s'étendent vers le bas, au-delà d'un point culminant (9) de la ligne de soudure (8) d'incurvation convexe.

7. Petite culotte jetable absorbante selon l'une des revendications qui précèdent, caractérisée en ce que la partie avant (2) et la partie arrière (3) sont formées séparément l'une de l'autre.

8. Petite culotte jetable absorbante selon la revendication 7, caractérisée en ce que la partie avant (2) et la partie arrière (3) sont soudées l'une à l'autre sur leurs faces opposées et dans la zone d'entrejambe, en suivant des lignes de soudure thermique ou par ultrasons, et en particulier de telle sorte que des bords étroits soient laissés sans soudure.

9. Petite culotte jetable absorbante selon l'une des revendications qui précèdent, caractérisée en ce que la garniture auxiliaire (15) présente des rabats latéraux extensibles partant de côtés latéraux opposés de cette dernière.

10. Petite culotte jetable absorbante selon l'une des revendications qui précèdent, caractérisée en ce que la couche de revêtement (6) et la couche extérieure (7) présentent des bords périphériques extérieurs qui dépassent vers l'extérieur au-delà du bord périphérique de la couche plus épaisse (8), et au niveau desquels elles sont en particulier collées ou soudées.
